# EUROPEAN PATENT APPLICATION

(11) **EP 2 881 116 A1**
(43) Date of publication of application: **10.06.2015**
(21) Application number: 14196368.6
(22) Date of filing: 04.12.2014
(51) Int. Cl.: A61K 31/426, A61K 9/16, A61K 9/28, A61K 9/20

(54) **Febuxostat composition**

(30) Priority: 05.12.2013 US 201314097394
(71) Applicant: Ranbaxy Laboratories Limited, New Delhi 110019 (IN)
(72) Inventor: Srivastava, Abhishek, 271001 Gonda, Uttar Pradesh (IN); Das, Surajit N., 700106 North 24 Parganas, West Bengal (IN); Garg, Mukesk Kumar, 122018 Gurgaon, Haryana (IN); Singla, Ajay Kumar, 122018 Gurgaon, Haryana (IN); Gupta, Amit, 122001 Gurgaon, Haryana (IN); Jadhav, Prakash A., 122001 Gurgaon (IN)
(74) Representative: Cronin, Brian Harold John

(57) **Abstract**

The present invention relates to an oral pharmaceutical composition of febuxostat which comprises an intragranular component and an extragranular component. Further, it relates to processes for the preparation of said composition and a method of using said composition.

## Description

### Cross-Reference to Related Application

This is a continuation-in-part of International Application No. PCT/IB2012/052890, with an international filing date of June 7, 2012, which claims priority to provisional Indian Patent Application No. 1664/DEL/2011, filed on June 13, 2011, and Indian Patent Application No. 410/DEL/2013, filed on February 13, 2013, the disclosures of which are both hereby incorporated by reference.

### Field of the Invention

The present invention relates to an oral pharmaceutical composition of febuxostat which comprises an intragranular component and an extragranular component. Further, it relates to processes for the preparation of said composition and a method of using said composition.

### Background of the Invention

Gout affects a large number of individuals worldwide and continues to increase in incidence. Gout includes a group of disorders including painful attacks of acute, monarticular, inflammatory arthritis due to uric acid crystals, deposition of urate crystals in joints, deposition of urate crystals in renal parenchyma, urolithiasis (formation of calculus in the urinary tract), and nephrolithiasis (formation of kidney stones).

The underlying metabolic aberration in gout is hyperuricemia. Hyperuricemia is associated with a serum uric acid (sUA) level of 6.8 mg/dL or greater, which is the upper limit of solubility of uric acid in extracellular fluids. However, hyperuricemia also has been associated with other levels of serum uric acid depending on factors such as gender and age, for example, hyperuricemia leads to gout when urate crystals are formed from supersaturated body fluids and deposited in joints, tophi, and parenchymal organs. In addition to gout, other disorders related to elevated serum uric acid levels include gout-associated inflammation, kidney stones, renal disorders, cardiovascular diseases, aberrant metabolic conditions, fatty liver disease, cognitive impairment, and dementia.

Methods for treating gout include the use of uric acid synthesis inhibitors to inhibit the accumulation of uric acid in the body. For example, use of xanthine oxidase inhibitors, such as febuxostat and allopurinol. Febuxostat is a non-purine selective inhibitor of xanthine oxidase that works by non-competitively blocking the channel leading to the active site on xanthine oxidase. Xanthine oxidase is needed to successively oxidize both hypoxanthine and xanthine to uric acid. Febuxostat inhibits xanthine oxidase, thereby reducing production of uric acid. For treatment of hyperuricemia in patients with gout, febuxostat is recommended at 40 mg or 80 mg once daily. No dose adjustment is necessary when administering febuxostat in patients with mild to moderate renal and hepatic impairment.

Febuxostat is commercially available under the brand name Uloric^{®} by Takeda in two strengths, 40 mg and 80 mg, in the form of film-coated tablets. The therapeutic dose required to be administered must thus be increased in order to compensate for this disadvantage.

The chemical name of febuxostat is 2-(3-cyano-4-isobutyloxyphenyl)-4-methyl-5-thiazolecarboxylic acid. It is disclosed in U.S. Patent No. 5,614,520. Febuxostat has the following chemical structure:

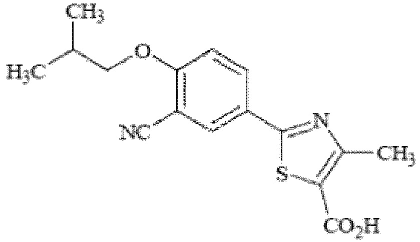

According to the Biopharmaceutics Classification System (BCS), febuxostat is classified as a Class II compound, exhibiting low solubility and high permeability. It suffers from the disadvantage of being poorly soluble in aqueous media, and as a result, having an undesirable dissolution profile, and consequently, poor bioavailability following oral administration.

U.S. Patent No. 6,225,474 discloses various polymorphs of febuxostat including an amorphous form and solvates (methanolate (D) and hydrate (G)). The polymorphs of febuxostat are designated as crystal forms A, B, C, D, and G. These polymorphs may interconvert from one to the other. U.S. Patent No. 6,225,474 discloses that if the crystal Forms B and D are exposed to air/moisture, then they may take water and convert into a hydrous form, *i.e.,* crystal G.

U.S. Patent No. 7,361,676 discloses solid oral dosage forms of febuxostat crystal Form A.

PCT Publication No. WO 2007/048332 discloses febuxostat composition and its application in the preparation of medicines for treating gout.

Indian Application No. 2116/MUM/2010 discloses compositions comprising febuxostat and a solubility enhancing agent. It further discloses that the solubility enhancing agent may be admixed with the drug or may be applied as a coating over the drug granules.

Indian Application No. 3620/CHE/2010 discloses an amorphous dispersion comprising febuxostat and a dispersing agent.

The present inventors have developed an oral pharmaceutical composition of febuxostat which is easy to manufacture and has an acceptable dissolution profile.

### Summary of the Invention

The present invention relates to an oral pharmaceutical composition of febuxostat comprising: (A) an intragranular component, and (B) an extragranular component, wherein the pharmaceutical composition is easy to manufacture and has an acceptable dissolution profile. The oral pharmaceutical composition of febuxostat comprises: (A) an intragranular component comprising: (i) an effective amount of febuxostat, (ii) wetting agent(s), (iii) diluent(s), (iv) binder(s), (v) disintegrant(s), and optionally (vi) glidant(s); and (B) an extragranular component comprising: (i) diluent(s), (ii) disintegrant(s), (iii) lubricant(s), and optionally (iv) glidant(s).

The present invention also includes processes for the preparation of said pharmaceutical composition, and a method of treating gout and hyperuricemia by administering said pharmaceutical composition.

### Detailed Description of the Invention

A first aspect of the present invention relates to an oral pharmaceutical composition of febuxostat comprising: (A) an intragranular component comprising: (i) an effective amount of febuxostat, (ii) wetting agent(s), (iii) diluent(s), (iv) binder(s), (v) disintegrant(s), and optionally (vi) glidant(s); and (B) an extragranular component comprising: (i) diluent(s), (ii) disintegrant(s), (iii) lubricant(s), and optionally (iv) glidant(s).

Embodiments of the composition may include one or more of the following features. The composition of the present invention may be in the form of a tablet or a capsule.

In one embodiment, the wetting agent is added to the granulating liquid. The wetting agent is present in an amount of more than 0.5% and less than 3% by weight of the composition, preferably in an amount of about 1% by weight of the composition.

In another embodiment, febuxostat is present in an amount ranging from about 2% to about 50%, preferably from about 5% to about 30%, more preferably from about 10% to about 20% by weight of the composition.

In another embodiment, the median particle size (D₅₀) of febuxostat ranges from about 0.1 µm to about 6 µm, preferably from about 1 µm to about 4 µm.

In another embodiment, a portion of the binder is blended with the drug, and the remaining portion is added to the granulating liquid. The ratio of the binder in the dry mix portion to that in the granulating liquid portion ranges from about 1:10 to about 10:1, preferably from about 1:5 to about 5:1.

In another embodiment, a portion of the disintegrant is present in the intragranular component and the remaining portion is added to the extragranular component. The disintegrant is present in an amount of about 5% to about 10% by weight of the composition. The ratio of the disintegrant in the intragranular portion to that in the extragranular portion ranges from about 1:10 to about 10:1, preferably from about 1:5 to about 5:1.

In another embodiment, the present invention relates to an oral pharmaceutical composition of febuxostat comprising: (A) an intragranular component comprising: (i) an effective amount of febuxostat, (ii) sodium lauryl sulfate as a wetting agent, (iii) a mixture of lactose and microcrystalline cellulose as diluents, (iv) hydroxypropyl cellulose as a binder, (v) croscarmellose sodium as a disintegrant, and (vi) a portion of colloidal silicon dioxide as a glidant; and (B) an extragranular component comprising: (i) microcrystalline cellulose as a diluent, (ii) croscarmellose sodium as a disintegrant, (iii) magnesium stearate as a lubricant, and (iv) the remaining portion of colloidal silicon dioxide as a glidant.

In another embodiment, the present invention relates to an oral pharmaceutical composition of febuxostat comprising: (i) about 10% to 20% by weight of febuxostat, (ii) more than 0.5% and less than 3% by weight of sodium lauryl sulfate, (iii) about 40% to 90% by weight of lactose and microcrystalline cellulose, (iv) about 1% to 3% by weight of hydroxypropyl cellulose, (v) about 5% to 10% by weight of croscarmellose sodium, (vi) about 0.01% to 2% by weight of colloidal silicon dioxide, and (vii) about 0.01 % to 4% by weight of magnesium stearate.

In another embodiment, the present invention relates to an oral pharmaceutical composition of febuxostat comprising: (A) an intragranular component comprising: (i) about 15.53% by weight of febuxostat, (ii) about 0.97% by weight of sodium lauryl sulfate, (iii) about 29.13% by weight of lactose and about 30.83% by weight of microcrystalline cellulose, (iv) about 1.94% by weight of hydroxypropyl cellulose, (v) about 5.83% by weight of croscarmellose sodium, and (vi) about 0.24% by weight of colloidal silicon dioxide; and (B) an extragranular component comprising: (i) about 9.71% by weight of microcrystalline cellulose, (ii) about 1.94% by weight of croscarmellose sodium, (iii) about 0.73% by weight of magnesium stearate, and (iv) about 0.24% by weight of silicon dioxide.

In another embodiment, the pharmaceutical composition of the present invention exhibits dissolution of at least 70% in 15 minutes and at least 90% in 30 minutes, when measured using USP type II (paddle) apparatus at 50 rpm in 900 mL of acetate buffer of pH 5.8 as the dissolution medium.

A second aspect of the present invention relates to an *in-vitro* dissolution method for the febuxostat composition, wherein the dissolution is performed according to USP dissolution method II (paddle apparatus, 50 rpm) employing 900 mL of acetate buffer of pH 5.8 as a dissolution media.

A third aspect of the present invention relates to a process of preparing an oral pharmaceutical composition of febuxostat comprising the steps of:
(a) blending the drug with the first portion of the binder and other intragranular excipients;
(b) preparing a binder solution by dispersing/dissolving the remaining portion of the binder in a granulating liquid;
(c) granulating the mixture of step (a) with the binder solution of step (b);
(d) drying the granules obtained in step (c) and adding the extragranular excipients to the dried granules; and
(e) compressing the granules of step (d) to form a tablet or filling the granules into a capsule.

A fourth aspect of the present invention relates to a process of preparing an oral pharmaceutical composition of febuxostat comprising the steps of:
(a) blending the drug with the first portion of the binder and other intragranular excipients;
(b) preparing a binder solution by dispersing/dissolving the remaining portion of the binder in a granulating liquid followed by the addition of a wetting agent;
(c) granulating the mixture of step (a) with the binder solution of step (b);
(d) drying the granules obtained in step (c) and adding the extragranular excipients to the dried granules; and
(e) compressing the granules of step (d) to form a tablet or filling the granules into a capsule.

A fifth aspect of the present invention provides a method of treating gout and hyperuricemia by administering to a person in need thereof an oral pharmaceutical composition of febuxostat comprising: (A) an intragranular component comprising: (i) an effective amount of febuxostat, (ii) wetting agent(s), (iii) diluent(s), (iv) binder(s), (v) disintegrant(s), and optionally (vi) glidant(s); and (B) an extragranular component comprising: (i) diluent(s), (ii) disintegrant(s), (iii) lubricant(s), and optionally (iv) glidant(s).

In a sixth aspect, the present invention relates to a stable, solid oral pharmaceutical composition comprising febuxostat wherein the composition is manufactured by wet granulation, which may be easy to manufacture and have an acceptable dissolution profile.

Embodiments of the composition may include one or more of the following features. The composition of the present invention may contain from about 1 mg to about 500 mg of febuxostat. Preferably, the composition contains from about 5 mg to about 200 mg of febuxostat. More preferably, the composition contains from about 10 mg to 140 mg of febuxostat. The febuxostat may have an average particle size of from 1 µm to 50 µm. Particularly, the average particle size of febuxostat ranges from 2 µm to 25 µm. The febuxostat may be of fine particle size, which has good solubility and is well suited for preparing pharmaceutical products.

In another embodiment, the composition comprises one or more pharmaceutically acceptable excipients selected from diluents, disintegrants, binders, lubricants, glidants, stabilizers, and organoleptic ingredients such as coloring agents, flavoring agents, sweeteners, and others known to the skilled person in the art. The composition can optionally be film-coated.

In a seventh aspect, the present invention relates to a process for preparing a stable, solid oral pharmaceutical composition of febuxostat comprising the steps of:
(a) blending a mixture of febuxostat and at least one pharmaceutically acceptable excipient;
(b) wetting the blend of step (a) with a granulating liquid;
(c) drying the wet granules of step (b) to obtain dried granules;
(d) optionally milling the dried granules of step (c), followed by adding at least one lubricant and optionally other pharmaceutically acceptable excipients to the dried milled granules; and
(e) compressing the lubricated granules of step (d) to form tablets or filling the lubricated granules into capsules.

The active ingredient and pharmaceutically acceptable excipient(s) are blended in a planetary mixer or a rapid mixer granulator. The granulating liquid includes, but is not limited to, water, ethanol, isopropyl alcohol, acetone, dichloromethane, and other hydroalcoholic solvents such as an isopropyl alcohol-water mixture.

The wet granulation process offers several advantages. This method assures content uniformity in the tablets and more uniform color distribution as compared to direct compression. The cohesiveness and compressibility of powders are improved because of the formulation of agglomerates (granules). The process prevents segregation or separation of components of a homogeneous powder mix during processing. High dose drugs having poor flow or compressibility properties can be prepared by wet granulation. Further, the dissolution rate of hydrophobic drugs may be improved by the wet granulation method.

In an eighth aspect, the invention relates to a method of treating gout and hyperuricemia by administering to a person in need thereof a stable, solid oral pharmaceutical composition comprising febuxostat and pharmaceutically acceptable excipients manufactured by wet granulation.

The pharmaceutical composition of febuxostat may be administered in combination with other therapeutic agents.

The term "pharmaceutical composition", as used herein, refers to the tablet or capsule composition of a predetermined quantity of active substance along with at least one pharmaceutically acceptable excipient.

The term "stable", as used herein, refers to a dosage form which is physically or polymorphically stable. The dosage form according to the present invention may remain physically stable, that is, that there are no substantial changes with respect to physical attributes such as color, etc. The dosage form according to the present invention may remain polymorphically stable, that is, the polymorph (crystalline or amorphous) in the dosage form does not convert into another form upon storage.

The term "about", as used herein, refers to any value which lies within the range defined by a variation of up to ±10% of the value.

The term "drug" or "active ingredient" or "febuxostat", as used herein, encompasses anhydrous form, hydrous form, different crystalline forms, amorphous form, solvates, or mixtures thereof, or any other form of febuxostat. Febuxostat is present in the present composition in an amount ranging from about 2% to about 50%, preferably from about 5% to about 30%, more preferably from about 10% to about 20% by weight of the composition. Febuxostat may be present in the present composition in an amount ranging from 5 mg to 500 mg. Preferably, the composition contains from about 5 mg to about 200 mg of febuxostat. More preferably, the composition contains from about 10 mg to about 140 mg of febuxostat.

Particle size can affect the solubility of a pharmaceutical compound. Particle size reduction can increase the dissolution rate and consequently the bioavailability. In the present invention, febuxostat is present in a micronized form having a median particle size (D₅₀) ranging from about 0.1 µm to about 6 µm, preferably from about 1 µm to about 4 µm.

The drug particles of the desired particle size may be obtained by any of the conventional processes known in the art such as mechanical milling, supercritical fluid processes, cryogenic spraying, or solvent evaporation. Mechanical reduction of particle size may be done by using a hammer mill, air jet mill, ball mill, or any other milling equipment known in the art. The particle size distribution of febuxostat particles of the present invention may be determined using an optical microscopic method; sedimentation techniques, for example, pipette analysis using an Andreassen pipette, sedimentation scale, photosedimentometer, or sedimentation in a centrifugal force field; pulse methods, for example using a Coulter counter; or sorting by means of gravitational or centrifugal force, sieve analysis, laser diffraction, or ultrasound attenuation spectroscopy. The particle size distribution of febuxostat particles of the present invention is particularly determined by laser diffraction using a Malvern^{®} Mastersizer^{®} laser diffraction instrument. The term "D₅₀ value" means that 50% of the particles have volume diameter of the specified value when measured by Malvern^{®} Mastersizer^{®} laser diffraction instrument.

The pharmaceutical compositions of the present invention may comprise one or more pharmaceutically acceptable excipients selected from diluents, disintegrants, binders, lubricants, glidants, wetting agents, and organoleptic ingredients such as coloring agents, flavoring agents, sweeteners, and others known to the skilled person in the art.

In the present invention, a wetting agent is present in the intragranular portion. The wetting agent may be blended with the drug or with the intragranular excipients, and/or may be added to the binder solution. Preferably, the wetting agent is added to the binder solution. The inventors of the present invention have found that the wetting agent, when added to the binder solution, gets uniformly dispersed to form a homogenous solution, and hence provides more intimate contact with the drug upon granulation, thus the wetting agent improves the dissolution of the active agent to a greater extent and is required in a lower amount. In contrast, if the wetting agent is directly mixed with the drug, the wetting agent would not be as effective and would be required in a larger amount.

The inventors of the present invention have further found that the amount of wetting agent to be used in the composition is a critical factor. Generally, the solubility of a poorly soluble drug increases with the amount of wetting agent. However, the inventors of the present invention have surprisingly found that compositions of the present invention containing a wetting agent in an amount of about 3% or more by weight of the composition, exhibit a slower dissolution profile. Without wishing to be bound by theory, the slower dissolution profile might be due to the enhancing effect of the wetting agent on the binding capacity of the binder.

Further, the compositions having a wetting agent in an amount of about 0.5% by weight of the composition exhibit a slower dissolution profile. The compositions having a wetting agent in an amount of more than about 0.5% and less than about 3% by weight of the composition give an optimum dissolution profile. The wetting agent is present in an amount of more than 0.5% and less than 3% by weight of the composition, preferably in an amount of about 1% by weight of the composition. The ratio of drug to wetting agent ranges from about 50:1 to about 1:1, preferably from about 20:1 to about 5:1, more preferably about 16:1.

Examples of wetting agents that can be used in the present invention include, but are not limited to, alkylglucosides; alkylmaltosides; lauryl macrogolglycerides; caprylocaproyl macrogolglycerides; polyoxyethylene alkyl ethers; polyoxyethylene alkylphenols; polyethylene glycol fatty acid esters; polyethylene glycol glycerol fatty acid esters; polyoxyethylene sorbitan fatty acid esters; polyoxyethylene-polyoxypropylene block copolymers; polyglycerol fatty acid esters; polyoxyethylene glycerides; polyoxyethylene vegetable oils; polyoxyethylene hydrogenated vegetable oils; sugar esters; sucroglycerides; alkyl ammonium salts; bile acids and salts; fatty acid derivatives of amino acids; lysophospholipids; phospholipids; salts of alkyl sulfates; alkyl ether sulfates and docusates; or mixtures thereof. Preferably, the wetting agent is sodium lauryl sulfate.

Suitable examples of diluents or fillers include, but are not limited to, microcrystalline cellulose, lactose such as lactose anhydrous or lactose monohydrate, sugar compressible, confectioners sugar, sucrose, glucose, maltose, calcium carbonate, calcium dihydrogen phosphate dihydrate, calcium phosphate-dibasic, calcium phosphate-tribasic, calcium sulfate, cellulose powdered, dextrin, dextrose, fructose, kaolin, lactitol, mannitol, sorbitol, starch, corn starch, or mixtures thereof. Preferably, the diluents comprise lactose monohydrate, microcrystalline cellulose, or mixtures thereof. Preferably, the two diluents used in the composition of the present invention are present in a ratio of 60:40. Further, the diluent(s) present in the intragranular portion are present in a ratio of about 1:1. Preferably, the diluent(s) present in the intragranular portion comprise a mixture of lactose monohydrate and microcrystalline cellulose, and the diluent present in the extragranular portion is microcrystalline cellulose. The diluent is present in an amount of about 40% to about 90% by weight of the composition.

In the present invention, a binder is present in the intragranular portion. A portion of the binder is blended with the active ingredient, and the other portion is dispersed or dissolved in the granulating liquid to form a binder solution. The particle size of the binder is important if the same is used in the dry mix before granulation to ensure uniform distribution of the binder. The ratio of binder in the dry mix portion to that in the granulating liquid ranges from about 1:10 to about 10:1, preferably about 1:5 to about 5:1. The binder is present in an amount of from about 0.01 % to about 10% by weight of the composition, preferably in an amount of from about 0.1 % to about 5% by weight of the composition, more preferably in an amount of from about 1 % to about 3% by weight of the composition. Suitable examples of binders include, but are not limited to, hydroxypropyl cellulose, pregelatinized starch, starch, povidone, carbomer, sodium carboxymethylcellulose, microcrystalline cellulose (MCC), micro fine cellulose, dextrin, lactose, glucose, guar gum, hypromellose, or mixtures thereof. Preferably, the binder is hydroxypropyl cellulose (HPC-L).

Examples of granulating liquids used in the present invention include, but are not limited to, water, ethanol, isopropyl alcohol, acetone, dichloromethane, or hydroalcoholic solvents such as an isopropyl alcohol-water mixture. The granulating liquid may include at least one pharmaceutically acceptable excipient. Particularly, the binder and wetting agent are a part of the granulating liquid.

In the present invention, a portion of the disintegrant is present in the intragranular component and the remaining portion is added to the extragranular component. The ratio of the disintegrant in the intragranular portion to that in the extragranular portion ranges from about 1:10 to about 10:1, preferably from about 1:5 to about 5:1. The inventors of the present invention have found that the composition having disintegrant in an amount of about 4.8% by weight of the composition gives a slower dissolution profile. The disintegrant is present in an amount of more than about 5%, preferably in an amount of about 5% to about 10%, more preferably in an amount of about 6% to about 8% by weight of the composition. Suitable examples of disintegrants include, but are not limited to, starch, sodium starch glycolate, croscarmellose sodium, crospovidone, alginic acid, low-substituted hydroxypropyl cellulose, carboxymethyl cellulose sodium, microcrystalline cellulose, calcium carbonate, sodium carbonate, agar, guar gum, or mixtures thereof. Preferably, the disintegrant is croscarmellose sodium.

In the present invention, a lubricant is present in an amount of about 0.01% to about 4% by weight of the composition. Suitable examples of lubricants include, but are not limited to, stearic acid, polyethylene glycol, magnesium stearate, calcium stearate, talc, zinc stearate, hydrogenated castor oil, silica, colloidal silica, cornstarch, calcium silicate, magnesium silicate, silicon hydrogel, or mixtures thereof.

In the present invention, a portion of glidant is present in the intragranular component, and the remaining portion is added to the extragranular component. A glidant, when added to the intragranular portion, aids in uniform mixing of the intragranular excipients. The glidant is present in an amount of about 0.01% to about 2% by weight of the composition. Suitable examples of glidants include, but are not limited to, colloidal silicon dioxide, colloidal silica, calcium silicate, magnesium silicate, colloidal silicon, silicon hydrogel, cornstarch, talc, or mixtures thereof.

The coloring agents and flavoring agents of the present invention may be selected from any FDA approved colors and flavors for oral use.

The composition of the present invention may be in the form of a tablet or a capsule. The composition may further be film-coated. Additional excipients such as film-coating polymers, solvents, plasticizers, antiadherents, opacifiers, and optionally colorants, pigments, antifoam agents, and polishing agents can be used in coatings.

Examples of film-coating polymers include, but are not limited to, cellulose derivatives such as methylcellulose, hydroxymethyl cellulose, hydroxyethyl cellulose, hydroxypropyl cellulose, hydroxymethylethyl cellulose, hydroxypropyl methylcellulose, sodium carboxymethyl cellulose, and ethyl cellulose; polymers based on carbohydrates and derivatives thereof; gums such as gum arabic and xanthan gum; alginates; vinyl polymers such as polyvinylpyrrolidones; acrylic polymers; waxes and fat substances; or mixtures thereof.

Alternatively, coating can be performed using any of the commercially available ready-to-coat preparations such as Opadry^{®} AMB, Opadry^{®} White, Opadry^{®} Clear, Opadry^{®} II, etc.

Suitable excipients used as adjuvants in the coating process include plasticizers, opacifiers, anti-adhesives, and polishing agents.

The composition of the present invention exhibits no significant difference in the rate and/or extent of absorption of febuxostat as compared to the marketed formulation of febuxostat available under the brand name Uloric^{®}.

The official media recommended for the dissolution of febuxostat tablets as per the USFDA is 0.05 M phosphate buffer of pH 6.0. As per the Summary Basis of Approval for Uloric^{®} tablets (USFDA), dissolution studies for febuxostat tablets have been carried out in 900 mL of 0.05M phosphate buffer of pH 6.8 using USP Apparatus II (paddle apparatus, 75 rpm). The inventors of the present invention have found that the phosphate buffer of pH 6.8 exhibits similar dissolution profiles for the compositions with or without wetting agents, and hence is unable to distinguish between the two formulations. However, the inventors have identified such an *in-vitro* dissolution media which can differentiate between the formulation, with or without wetting agents, by giving distinct dissolution profiles. The differentiating dissolution medium is an acetate buffer of pH 5.8. When dissolution is carried out using dissolution method II (paddle apparatus, 50 rpm) using 900 mL of acetate buffer ofpH 5.8 as the dissolution media, the formulations with or without the wetting agent give distinct dissolution profiles clearly highlighting the difference between the two formulations.

The composition of the invention may be used in treating conditions such as gout and hyperuricemia. The pharmaceutical composition of febuxostat may be administered in combination with other therapeutic agents.

The present invention is illustrated below by reference to the following examples. However, one skilled in the art will appreciate that the specific methods and results discussed are merely illustrative of the invention, and not to be construed as limiting the invention.

### EXAMPLES

### Example 1:

| **Ingredients** | **Percent w/w** |
|---|---|
| ***Intragranular*** | |
| Febuxostat | 15.53 |
| Lactose | 29.13 |
| Microcrystalline Cellulose | 30.83 |
| Hydroxypropyl Cellulose (Dry Mix Portion) | 1.46 |
| Hydroxypropyl Cellulose (Binder Portion) | 0.48 |
| Croscarmellose Sodium | 5.83 |
| Colloidal Silicon Dioxide | 0.24 |
| Sodium Lauryl Sulfate | 0.97 |
| Purified Water | q.s. |

| ***Extragranular*** | |
|---|---|
| Microcrystalline Cellulose | 9.71 |
| Croscarmellose Sodium | 1.94 |
| Colloidal Silicon Dioxide | 0.24 |
| Magnesium Stearate | 0.73 |

| ***Film Coating*** | |
|---|---|
| Opadry^{®} II | 2.91 |
| Purified Water | q.s. |

### Procedure:

1. Febuxostat and lactose were sifted together through a suitable mesh.
2. Microcrystalline cellulose, croscarmellose sodium (intragranular), colloidal silicon dioxide (intragranular), and hydroxypropyl cellulose (dry mix portion) were sifted together through a suitable mesh.
3. Extragranular microcrystalline cellulose, croscarmellose sodium, and colloidal silicon dioxide were sifted together through a suitable mesh.
4. A binder solution was prepared by dispersing the second portion of hydroxypropyl cellulose (binder portion) in water under mechanical stirring.
5. Sodium lauryl sulfate was dissolved into the solution of step 4 under mechanical stirring.
6. The sifted materials of step 1 and step 2 were loaded into the bowl of a rapid mixer granulator, and dry mixing was carried out.
7. The material of step 6 was granulated using the binder solution of step 5.
8. The granules obtained in step 7 were dried in a suitable dryer and milled.
9. The extragranular material of step 3 was added to the granules of step 8 and blended.
10. The blend from step 9 was lubricated with magnesium stearate.
11. The blend of step 10 was compressed into tablets with suitable tooling, and the tablets were coated with a suspension of Opadry^{®} II.

### Example 2:

| **Ingredients** | **Percent w/w** |
|---|---|
| ***Intragranular*** | |
| Febuxostat | 15.53 |
| Microcrystalline Cellulose | 31.07 |
| Lactose Monohydrate | 29.13 |
| Croscarmellose Sodium | 5.83 |
| Colloidal Silicon Dioxide | 0.24 |
| Hydroxypropyl Cellulose (HPC-L) | 1.94 |
| Sodium Lauryl Sulfate | 0.97 |
| Purified Water | q.s. |

| ***Extragranular*** | |
|---|---|
| Microcrystalline Cellulose | 9.71 |
| Croscarmellose Sodium | 1.94 |
| Colloidal Silicon Dioxide | 0.24 |
| Magnesium Stearate | 0.49 |

| ***Film Coating*** | |
|---|---|
| Opadry^{®} II | 2.91 |
| Purified Water | q.s. |

### Procedure:

The tablets of Example 2 were prepared by following the same procedure as Example 1.

### Table 1: Dissolution study

Table 1 provides dissolution data of febuxostat tablets prepared as per Example 2 wherein the median particle size D₅₀ of febuxostat is 1.8 µm. For determination of drug release rate, USP type II (paddle apparatus, 75 rpm) was used wherein 900 mL of phosphate buffer of pH 6.8 was used as the dissolution medium.

**Table 2: Stability study**

| **Time (minutes)** | **Percent Drug Release** |
|---|---|
| 10 | 100 |
| 15 | 100 |
| 30 | 100 |

The tablets prepared as per Example 2 were packed into blister packs and were subjected to stability studies at 40°C/75% RH over a period of 6 months. The results of the stability study are provided below:

| **Storage Conditions** | **40°C/75% RH** | | |
|---|---|---|---|
| | **Initial** | **3 months** | **6 months** |
| **Total Related Substances (% w/w)** | 0.01 | 0.03 | 0.03 |

The above stability data shows that the composition is stable even after storage for about 6 months.

### Example 3:

| **Ingredients** | **Percent w/w** |
|---|---|
| ***Intragranular*** | |
| Febuxostat | 15.53 |
| Microcrystalline Cellulose | 30.10 |
| Lactose Monohydrate | 28.16 |
| Croscarmellose Sodium | 7.77 |
| Colloidal Silicon Dioxide | 0.24 |
| Hydroxypropyl Cellulose (HPC-L) | 1.94 |
| Sodium Lauryl Sulfate | 1.94 |
| Purified Water | q.s. |

| ***Extragranular*** | |
|---|---|
| Microcrystalline Cellulose | 8.74 |
| Croscarmellose Sodium | 1.94 |
| Colloidal Silicon Dioxide | 0.24 |
| Magnesium Stearate | 0.49 |

| ***Film Coating*** | |
|---|---|
| Opadry^{®} II | 2.91 |
| Purified Water | q.s. |

### Procedure:

The tablets of Example 3 were prepared by following the same procedure as Example 1.

### Table 3: Dissolution study

Table 3 provides dissolution data of febuxostat tablets prepared as per Example 3 wherein the median particle size D₅₀ of febuxostat is 2.46 µm. For determination of drug-release rate, USP type II (paddle apparatus, 75 rpm) was used wherein 900 mL of phosphate buffer of pH 6.8 was used as the dissolution medium.

| **Time (minutes)** | **Percent Drug-Release** |
|---|---|
| 10 | 99 |
| 15 | 99 |
| 30 | 99 |

### Example 4:

| **Ingredients** | **Percent w/w** |
|---|---|
| ***Intragranular*** | |
| Febuxostat | 16.00 |
| Lactose Monohydrate | 15.30 |
| Microcrystalline Cellulose | 49.75 |
| Hydroxypropyl Cellulose | 2.00 |
| Croscarmellose Sodium | 3.00 |
| Sodium Lauryl Sulfate | 1.00 |
| Colloidal Silicon Dioxide | 0.25 |
| Purified Water | q.s. |

| ***Extragranular*** | |
|---|---|
| Croscarmellose Sodium | 2.00 |
| Microcrystalline Cellulose | 10.00 |
| Colloidal Silicon Dioxide | 0.20 |
| Magnesium Stearate | 0.50 |

### Procedure:

The tablets of Example 4 were prepared by following the same procedure as Example 1.

### Reference Example 1: (Tablets without wetting agent)

| **Ingredients** | **Percent w/w** |
|---|---|
| ***Intragranular*** | |
| Febuxostat | 16.00 |
| Lactose Monohydrate | 15.30 |
| Microcrystalline Cellulose | 60.00 |
| Hydroxypropyl Cellulose | 3.00 |
| Croscarmellose Sodium | 3.00 |
| Purified Water | q.s. |

| ***Extragranular*** | |
|---|---|
| Croscarmellose Sodium | 2.00 |
| Colloidal Silicon Dioxide | 0.20 |
| Magnesium Stearate | 0.50 |

### Procedure:

1. Febuxostat and lactose monohydrate were sifted together through a suitable mesh.
2. Microcrystalline cellulose, croscarmellose sodium (intragranular), and hydroxypropyl cellulose (dry mix portion) were sifted together through a suitable mesh.
3. Extragranular croscarmellose sodium and colloidal silicon dioxide were sifted together through a suitable mesh.
4. A binder solution was prepared by dispersing the second (binder) portion of hydroxypropyl cellulose in water under mechanical stirring.
5. The sifted materials of step 1 and step 2 were loaded into the bowl of a rapid mixer granulator and dry mixing was carried out.
6. The material of step 5 was granulated using the binder solution of step 4.
7. The granules obtained in step 6 were dried in a suitable dryer and milled.
8. The extragranular material of step 3 was added to the granules of step 7 and blended.
9. The blend from step 8 was lubricated with magnesium stearate and was compressed into tablets with suitable tooling.

### Table 4: Dissolution study in 900 mL of phosphate buffer of pH 6.8 as the dissolution medium

Table 4 provides dissolution data of febuxostat tablets prepared as per Example 4, Reference Example 1, and reference formulation (Uloric^{®} film-coated tablet 80 mg). For determination of the drug release rate, USP type II (paddle apparatus, 75 rpm) was used wherein 900 mL of phosphate buffer ofpH 6.8 was used as the dissolution medium.

| **Time (minutes)** | **Percent Drug Release** | | |
|---|---|---|---|
| | **Example 4** | **Reference Example 1** | **Uloric^{®} Tablet** |
| 15 | 98 | 101 | 102 |
| 30 | 98 | 103 | 103 |

The results show that both the compositions of Example 4 (with the wetting agent) and Reference Example 1 (without the wetting agent) show similar dissolution, even at 15 minutes interval, when the dissolution is carried out in 900 mL of phosphate buffer of pH 6.8.

### Table 5: Dissolution study in 900 mL of acetate buffer of pH 5.8 as the dissolution medium

Table 5 provides dissolution data of febuxostat tablets prepared as per Example 4, Reference Example 1, and reference formulation (Uloric^{®} film-coated tablet 80 mg). For determination of the drug release rate, USP type II (paddle apparatus, 50 rpm) was used wherein 900 mL of acetate buffer ofpH 5.8 was used as the dissolution medium.

| **Time (minutes)** | **Percent Drug Release** | | |
|---|---|---|---|
| | **Example 4** | **Reference Example 1** | **Uloric^{®} Tablet** |
| 10 | 70 | 67 | 80 |
| 15 | 74 | 72 | 88 |
| 30 | 92 | 76 | 94 |

The results show that the compositions of Example 4 (with wetting agent) and Reference Example 1 (without the wetting agent) show distinct dissolution profiles when the test is carried out in 900 mL of acetate buffer of pH 5.8 as the dissolution medium, clearly highlighting the difference between the two formulations.

### Example 5:

Example 5 is directed to a wet granulation process for making febuxostat tablets.

| **Ingredients** | **Percent w/w** |
|---|---|
| Febuxostat # | 15.38 |
| Lactose | 35.66 |
| Microcrystalline Cellulose | 35.57 |
| Hydroxypropyl Cellulose | 3.08 |
| Purified Water | q.s. |
| Sodium Croscarmellose | 5.00 |
| Colloidal Silicon Dioxide | 0.96 |
| Magnesium Stearate | 1.44 |
| **Core Tablet** | **97.09** |
| Opadry^{®} II | 2.91 |
| Purified Water | q.s. |

| | |
|---|---|
| # Average particle size <50.00 micron | |

### Procedure:

1. Febuxostat, lactose and microcrystalline cellulose were sifted through a suitable mesh.
2. Hydroxypropyl cellulose was sifted through a suitable mesh and dissolved in purified water.
3. The blend from Step 1 was granulated with the binder solution of Step 2.
4. Granules obtained in Step 3 were dried in a suitable dryer.
5. Sodium croscarmellose and colloidal silicon dioxide were sifted through a suitable mesh.
6. The granules obtained in Step 4 were blended with the mixture of Step 5.
7. The blend from Step 6 was lubricated with magnesium stearate.
8. The blend of Step 7 was compressed into tablets with suitable tooling, and the tablets were coated with a suspension of Opadry^{®} II.

While several specific embodiments of the invention have been illustrated and described, it will be apparent to a person skilled in the art that various modifications and combinations of the invention detailed in the text can be made without departing from the spirit and scope of the invention.

## Claims

1. An oral pharmaceutical composition of febuxostat comprising:
(A) an intragranular component comprising: (i) an effective amount of febuxostat, (ii) wetting agent(s), (iii) diluent(s), (iv) binder(s), (v) disintegrant(s), and optionally (vi) glidant(s); and
(B) an extragranular component comprising: (i) diluent(s), (ii) disintegrant(s), (iii) lubricant(s), and optionally (iv) glidant(s)
wherein the intragranular component comprises a wetting agent and a granulating liquid and the wetting agent is added to the granulating liquid.

2. The pharmaceutical composition according to claim 1, wherein the wetting agent is present in an amount of more than 0.5% and less than 3% by weight of the composition.

3. The pharmaceutical composition according to claim 1, wherein febuxostat is present in an amount of from about 2% to about 50% by weight of the composition.

4. The pharmaceutical composition according to claim 1, wherein the median particle size (D₅₀) of febuxostat ranges from about 0.1 µm to about 6 µm.

5. The pharmaceutical composition according to claim 4, wherein the median particle size (D₅₀) of febuxostat ranges from about 1 µm to about 4 µm.

6. The pharmaceutical composition according to claim 1, wherein a portion of the binder is blended with the drug and the remaining portion is added to the granulating liquid.

7. The pharmaceutical composition according to claim 1, wherein the ratio of binder in the dry mix portion to that in the granulating liquid portion ranges from about 1:10 to about 10:1.

8. The pharmaceutical composition according to claim 1, wherein the disintegrant is present an amount of from about 5% to about 10% by weight of the composition.

9. The pharmaceutical composition according to claim 1, comprising:
(A) an intragranular component comprising: (i) an effective amount of febuxostat, (ii) sodium lauryl sulfate as the wetting agent, (iii) mixture of lactose and microcrystalline cellulose as diluents, (iv) hydroxypropyl cellulose as the binder, (v) croscarmellose sodium as the disintegrant, and (vi) colloidal silicon dioxide as the glidant; and
(B) an extragranular component comprising: (i) microcrystalline cellulose as the diluent, (ii) croscarmellose sodium as the disintegrant, (iii) magnesium stearate as the lubricant, and (iv) remaining portion of colloidal silicon dioxide as the glidant.

10. The pharmaceutical composition according to claim 1, comprising: (i) about 10% to 20% by weight of febuxostat, (ii) about 0.5% to less than 3% by weight of sodium lauryl sulfate, (iii) about 40% to 90% by weight of lactose and microcrystalline cellulose, (iv) about 1 % to 3% by weight of hydroxypropyl cellulose, (v) about 5% to 10% by weight of croscarmellose sodium, (vi) about 0.01% to 2% by weight of colloidal silicon dioxide, and (vii) about 0.01% to 4% by weight of magnesium stearate.

11. The pharmaceutical composition according to claim 1, comprising:
(A) an intragranular component comprising: (i) about 15.53% by weight of febuxostat, (ii) about 0.97% by weight of sodium lauryl sulfate, (iii) about 29.13% by weight of lactose and about 30.83% by weight of microcrystalline cellulose, (iv) about 1.94% by weight of hydroxypropyl cellulose, (v) about 5.83% by weight of croscarmellose sodium, and (vi) about 0.24% by weight of colloidal silicon dioxide; and
(B) an extragranular component comprising: (i) about 9.71% by weight of microcrystalline cellulose, (ii) about 1.94% by weight of croscarmellose sodium, (iii) about 0.73% by weight of magnesium stearate, and (iv) about 0.24% by weight of silicon dioxide.

12. The pharmaceutical composition according to claim 1, having a dissolution of at least 70% in 15 minutes and at least 90% in 30 minutes, when measured using USP type II (paddle apparatus, 50 rpm) in 900 mL of acetate buffer of pH 5.8 as the dissolution medium.

13. An *in-vitro* dissolution method for a febuxostat composition, wherein the dissolution is performed according to USP dissolution method II (paddle apparatus, 50 rpm) employing 900 mL of acetate buffer of pH 5.8 as the dissolution media.

14. A process of preparing an oral pharmaceutical composition of febuxostat comprising (A) an intragranular component of (i) an effective amount of febuxostat, (ii) one or more wetting agents, (iii) one or more diluents, (iv) one or more binders, (v) one or more disintegrants, and optionally (vi) one or more glidants; and (B) an extragranular component of (i) one or more diluents, (ii) one or more disintegrants, (iii) one or more lubricants and optionally (iv) one or more glidants, wherein the intragranular component comprises a granulating liquid, and wherein the process comprises the steps of:
(a) blending the febuxostat with a first portion of a binder and other intragranular excipients;
(b) preparing a binder solution by dispersing/dissolving the remaining portion of the binder to the granulating liquid;
(c) granulating the mixture of step (a) with the binder solution of step (b);
(d) drying the granules obtained in step (c), and adding the extragranular excipients to the dried granules; and
(e) compressing the granules of step (d) to form a tablet or filling the granules into a capsule.

15. The process of claim 14, wherein the wetting agent is added to the binder solution of the binder and the granulating liquid of step (b).

16. A method of treating gout and/or hyperuricemia by orally administering to a person in need thereof the oral pharmaceutical composition of febuxostat according to claim 1.
